# EUROPEAN PATENT APPLICATION

(11) **EP 1 342 489 A1**
(43) Date of publication of application: **10.09.2003**
(21) Application number: 03250792.3
(22) Date of filing: 07.02.2003
(51) Int. Cl.: A61P 5/02, A61P 25/20, A61M 21/00

(54) **Method of affecting sleep and sleep-related behaviors**

(30) Priority: 08.02.2002 GB 0203045
(71) Applicant: Johnson & Johnson Consumer Companies, Inc., Skillman, New Jersey 08858 (US)
(72) Inventor: McCulloch, Laura, King's Somborne, Hampshire S020 (GB); Wiegand, Benjamin Carl, Newtown, Pennsylvania 18940 (US)
(74) Representative: Mercer, Christopher Paul

(57) **Abstract**

A method of affecting sleep and sleep-related behaviors of a mammal having a diurnal rhythm, by reducing the basal activity of the hypothalamus-pituitary-adrenal axis by administering an effective amount of a sensory regimen is disclosed. Such reduction may be accomplished by reducing at least one of the following:
a. the average total daily amount of adrenocortical hormone; or
b. the average total daily amount adrenocortical hormone minus the integrative measure of morning peak adrenocortical hormone.

Preferably, such reduction also includes reducing at least one of the following:
c. the level of adrenocortical hormone 4 hours to 8 hours after waking;
d. the level of adrenocortical hormone in the period of time preceding bedtime; or
e. the level of adrenocortical hormone below the onset of sleep threshold.

## Description

### FIELD OF THE INVENTION

The invention relates to a method of affecting sleep and sleep-related behaviors. More particularly, the invention relates to a method of affecting sleep and sleep-related behaviors by reducing the basal activity of the hypothalamus-pituitary-adrenal axis.

### BACKGROUND OF THE INVENTION

It has been recognized that sleep and sleep-related behaviors may be affected by the hypothalamus-pituitary-adrenal (HPA) axis. The reactivity of the HPA axis may be monitored by measuring the level of adrenocortical hormones. An adrenocortical hormone that can be easily measured is cortisol, which can be found in the blood and the saliva of human beings. Cortisol is produced in the adrenal cortex and is involved in a number of neurological events.

Cortisol secretion follows a diurnal rhythm. Essentially, for mammals following a 24 hour day divided into two main periods -- one a period of wakefulness and the other a period of sleepfulness -- cortisol production peaks approximately 30 to 45 minutes following morning waking, and then declines throughout the day, reaching a minimum in the hours preceding bedtime. Throughout nighttime sleep, cortisol is secreted in a pulsatile style.

"The Hypothalamic-Pituitary-Adrenocortical System and Sleep in Man" by Freiss *et al.* in *Advances in Neuroimmunology* 1995, Volume 5, 111-125 discloses that the hormones of the HPA axis may affect sleep. Corticotropin releasing hormone (CRH) and vasopressin are reported to reduce the slow wave sleep and rapid eye movement phases of sleep, leading to a shallower sleep and increased wakefulness. The effects of steroids, including cortisol, are reported in this review as being less well understood with no causal or temporal link of cortisol secretion and sleep architecture having been established. There is no discussion of the effect of the HPA axis in the hours prior to bedtime on sleep.

Reduction in the level of adrenocortical hormones has been demonstrated to be effective in promoting improved sleep behavior. For example, U.S. Patent Application Serial No. 09/676,876, filed September 29, 2000 entitled "Method For Calming Human Beings Using Personal Care Compositions" discloses a method to calm humans and improve sleep behavior over the short-term by reducing the level of adrenocortical hormone at the time of administering personal care compositions, particularly in those aged 1 day to 12 years, when practiced immediately prior to bedtime. The reduction in the level of adrenocortical hormone demonstrated is not sustained sufficiently to reset the basal level of the adrenocortical hormones.

Reduction in the level of adrenocortical hormones has also been demonstrated to be effective in reducing stress response. For example, Japanese Kokai 9-227399 discloses a method of reducing adrenocortical hormone level to reduce stress response by inhaling essences of the family of *labiatae* plants. The method reduces the hormone level only over the short-term and is not disclosed to affect sleep.

One of the main deficiencies with each of the above-described methods and multitude of other methods employing, *inter alia,* folk remedies and herbal treatments, is that none are sufficient to reduce the basal activity of the hypothalamus-pituitary-adrenal axis and, thus, only provide short-term improvement to sleep and sleep-related behaviors. The methods of the invention provide a long-term improvement to sleep and sleep-related behaviors by reducing the basal activity of the HPA axis of the mammal.

### SUMMARY OF THE INVENTION

The invention relates to a method of affecting sleep-related behavior in a mammal, comprising the step of reducing the basal activity of the HPA axis of the mammal, wherein the reducing step preferably includes administering an effective amount of a sensory regimen to mammal.

### BRIEF DESCRIPTION OF THE DRAWINGS

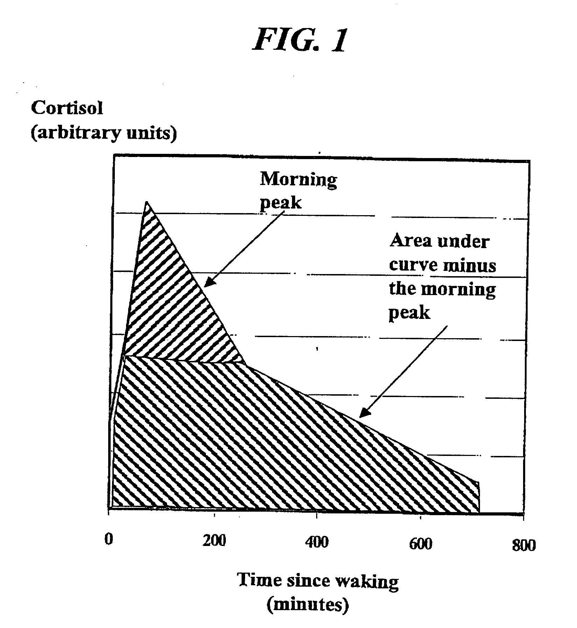

Figure 1 is a graph illustrating the level of adrenocortical hormone as a function of time for a mammal over a wakeful period of its diurnal rhythm, including the total daily amount of adrenocortical hormone; the integrative measure of morning peak adrenocortical hormone; and the onset of sleep threshold.

### DETAILED DESCRIPTION OF THE INVENTION

The invention relates to a method of affecting sleep and sleep-related behaviors in a mammal having a diurnal rhythm, comprising the step of reducing the basal activity of the HPA axis of the mammal, wherein the reducing step, includes administering an effective amount of a sensory regimen to the mammal.

As used herein, "sleep-related behavior" shall include the ease of onset of sleep, quality of sleep, incidence of early awakenings and satisfaction with sleep.

As used herein, "HPA axis" shall mean the hypothalamus-pituitary-adrenal axis, which is an endocrine system which affects several physiological functions as described by George P. Chrousos and Philip W. Gold in "The Concepts of Stress and Stress System Disorders -- Overview of Physical and Behavioral Homeostasis," *JAMA,* March 4, 1992, Volume 267, Number 9. The effect of the HPA axis on sleep has been explored in "The Hypothalamic-Pituitary-Adrenocortical System and Sleep in Man" by Freiss *et al.* in *Advances in Neuroimmunology* 1995, Volume 5, 111-125.

As used herein, "basal activity of the HPA axis" shall mean the baseline activity level of the HPA axis in a mammal.

As used herein, "mammals" shall include any of a class of warm-blooded higher vertebrates that nourish their young with milk secreted by mammary glands and have skin usually more or less covered with hair, and non-exclusively includes humans, dogs and cats.

As used herein, "effective amount" refers to the frequency, level and duration of the regimen of sensory experience sufficient to significantly induce a positive modification in the condition to be treated, but low enough to avoid serious side effects (at a reasonable benefit/risk ratio), within the scope of sound medical judgment. The effective amount of the compound or composition will vary with the particular condition being treated, the age and physical condition of the patient being treated, the severity of the condition, the frequency, level and duration of the treatment, the nature of concurrent therapy, the specific compound or composition employed, the particular pharmaceutically-acceptable carrier utilized, and like factors within the knowledge and expertise of the attending physician. Use of a multiple sensory regimen can affect the duration that would be needed to create the desired response.

In one embodiment, the basal activity of the HPA axis is reduced by reducing the average total daily amount of adrenocortical hormone in the mammal. In another embodiment, the basal activity of the HPA axis is reduced by reducing the average total daily amount of adrenocortical hormone minus the integrative measure of morning peak adrenocortical hormone in the mammal. Preferably, the adrenocortical hormone measured is cortisol.

In preferred embodiments, the basal activity of the HPA axis is reduced by reducing at least one of the following:
a. the level of adrenocortical hormone in the mammal 4 to 8 hours after waking;
b. the level of adrenocortical hormone in the mammal in the period of time preceding bedtime; or
c. the level of adrenocortical hormone in the mammal below said onset of sleep threshold.

In another embodiment, the invention is directed to a method of affecting sleep and sleep-related behaviors in a mammal having a diurnal rhythm, including at least one step selected from the group consisting of:
a. reducing the level of adrenocortical hormone in the mammal 4 hours to 8 hours after waking;
b. reducing the level of adrenocortical hormone in the mammal in the period of time preceding bedtime; and
c. reducing the level of adrenocortical hormone in the mammal below said onset of sleep threshold.

Preferably, the average total daily amount of adrenocortical hormone over a 24-hour period in the mammal is reduced by at least about 5% to about 50%, more preferably by at least about 10% to about 40%, and most preferably by at least about 15% to about 30%, based on the total daily amount of adrenocortical hormone present in the mammal at the start of the regimen.

Preferably, the average total daily amount adrenocortical hormone minus said integrative measure of morning peak adrenocortical hormone over a 24-hour period in the mammal is reduced by at least about 5% to about 70%, more preferably by at least about 10% to about 60%, and most preferably by at least about 20% to about 50%, based on the total daily amount of adrenocortical hormone minus said integrative measure of morning peak adrenocortical hormone present in the mammal at the start of the regimen.

Preferably, the level of adrenocortical hormone in the mammal 4 hours to 8 hours after waking is reduced by at least about 5% to about 70%, more preferably by at least about 10% to about 60%, and most preferably by at least about 20% to about 50%, based on the level of adrenocortical hormone present during that same time period in the mammal at the start of the regimen.

Preferably, the level of adrenocortical hormone in the mammal in the period preceding bedtime, preferably about 4 hours preceding bedtime, is reduced by at least about 3% to about 50%, more preferably by at least about 5% to about 30%, and most preferably by at least about 5% to about 20%, based on the level of adrenocortical hormone present during that same time period in the mammal at the start of the regimen.

Preferably, the level of adrenocortical hormone in the mammal is reduced to less than 0.3 micrograms/deciliter, more preferably to less than 0.2 micrograms/deciliter, and most preferably to less than 0.15 micrograms/deciliter.

In the method of the invention, the sensory regimen may further include at least one of the following steps selected from the group consisting of:
a. administering at least one corticotropic-releasing hormone (CRH) antagonist;
b. administering at least one anti-depressant; or
c. administering at least one pharmacalogical sleep aid.

Preferably, the basal activity of the HPA axis of the mammal is reduced within a period of 2 days to 14 days from the start of said regimen.

To measure the basal activity of the HPA axis, cortisol levels in the body, including cortisol found in the serum, saliva or urine, may be measured. Preferably, the cortisol level in saliva is measured because:
(1) collecting saliva is the least stressful, least painful and least invasive;
(2) cortisol levels in saliva are representative of a mammal's normal response;
(3) cortisol in saliva is not bound and thus is a more accurate predictor of physiological effect; and
(4) measurement of cortisol in saliva is more instantaneous (less cumulative) relative to other bodily fluids, such as urine.

As described in co-pending US provisional patent application 60/256,812, an enzyme linked immunoassay (ELISA) methodology is useful in the measurement of cortisol at the concentrations typically found in the saliva of a mammal.

Adrenocortical hormones, including cortisol, follow a diurnal rhythym over a 24-hour period with a wakeful period and sleepful period. The area under the curve of the daytime profile can be considered as having two distinct areas, the morning peak (referred to herein, as "integrative measure of morning peak adrenocortical hormone")(typically occurring 30 to 45 minutes following waking) and the remaining area under curve. These areas are represented in Figure 1. The area under the curve (referred to herein, as "total daily amount of adrenocortical hormone") minus the peak area (integrative measure of morning peak adrenocortical hormone) is yet another useful index of basal level of the HPA axis. Furthermore, the level of adrenocortical hormone in the mammal 4 to 8 hours after waking; the level of adrenocortical hormone in the mammal in the period of time preceding bedtime; and the level of adrenocortical hormone at the onset of sleep threshold are also shown in Figure 1.

The sensory regimen useful in the method of the invention is any regimen that is relaxing to the user. Stimuli used to provide the sensory experience generally are those that provide an experience that the individual who intends to practice the invention finds pleasant. Generally, the sensory regimen is selected from the group consisting of auditory stimuli, visual stimuli, tactile stimuli, gustatory stimuli and olfactory stimuli, and combinations thereof.

Auditory stimuli useful in the method of the invention include, but are but are not limited to, music and sounds of nature that are soothing or relaxing to the user. The term music is used herein to include instrumental and lyrical compositions; tunes; melodies; harmonies; songs; beats and frequencies such as those from metronomes, tuning forks, bells, beat machines, chimes; poetry and rhymes. The music may be of any genre, including, but not limited to, classical, soft rock, easy listening, progressive, country, and show tunes. The sounds of nature include, but are not limited to, animal sounds, such as whales singing or birds chirping; insect sounds, such as crickets; and sounds of the environment, such as a running stream or a waterfall. Sounds that have consistently soft dynamics with minimal melodic and harmonic variability, having little or no conventional beat pitch, little or no vocal, slow tempo, little or no percussion or strong rhythm are particularly effective in relaxing or soothing the user. Sounds that use a binaural beat created by using two pure frequencies, usually one in each ear, are useful in improving the mood of the user. Binaural beats in the frequency range of delta, theta and alpha brain wave frequencies are useful for relaxing the user and beats in the frequency range of beta wave activity are useful for promoting mental alertness in the user. The auditory stimuli may include, but are not limited to, a cassette tape, videotape, compact disc, interactive toys and games, websites, and a computer audio file.

Visual stimuli useful in the method of the invention include, but are not limited to, soft lights, candles, videos, movies, paintings, murals, books, landscapes, interactive toys and games, websites, and computer image files that are soothing or relaxing to the user. The soft lights may be of any color, such as blue, green, pink, purple, and the like. Cool colors, such as blue and green hues, are preferred to soothe the user and aid relaxation; and warmer colors, such as oranges and reds are preferred to uplift the user. Pastel shades, which are low saturation hues, are useful in soothing the user. The light may be provided in the kit as a bulb, which can be inserted into a lamp at home, or may be provided in the kit as a lamp. Lights that utilize fiber optics may also be useful in the kits of this invention. The fiber optic lights may, as is known in the art, change colors intermittently. Soft lighting of approximately 500 lux is useful in relaxing the user, particularly in the evening hours preceding bedtime. Bright light of around 2000 lux or greater is useful in improving the mood of the user when used in the wakeful period of the day such as at awakening or any other time during the day prior to the few hours preceding bedtime.

Combinations of light and sound that have frequency patterns in the range of delta, theta and alpha brain wave frequencies are useful for relaxing the user and those that have patterns in the frequency range of beta wave activity are useful for promoting mental alertness in the user.

Tactile stimuli useful in the method of the invention include, but are not limited to, computer software, interactive toys and games, bubble baths, lotions, and personal care compositions.

The computer software may be of an interactive nature, such that the consumer relaxes while utilizing the software. Such software includes video games, crossword puzzles and the like.

Gustatory stimuli useful in the method of the present invention include food and beverages, such as, but not limited to, fruits, candies, crackers, cheese, teas, and the like. Olfactory stimuli useful in the method of the invention include sensory experiences, such as fragrances. Fragrances that the user finds pleasant and have a calming effect on their mood are useful in the practice of this invention. Suitable fragrances include, but are not limited to, those perfume compositions described in UK application 0031047.4 (now International Publication Number WO 02/49600 Al such as PD 1861 available from Quest International. Also suitable are the fragrances described in co-pending U.S. Patent Application Serial No. 09/676,876, filed September 29, 2000 entitled "Method For Calming Human Beings Using Personal Care Compositions," the disclosure of which is incorporated herein by reference. Generally, the fragrance may be any fragrance that is perceivable and relaxing to the user and will reduce the activity of the HPA axis.

A preferred means of delivering sensory stimuli is in the form of a personal care composition. Personal care compositions are particularly useful in delivering olfactory stimuli. For example, the sensory fragrance may be produced by blending the selected essential oils and odoriferous components under ambient conditions until the final mixture is homogenous using equipment and methodology commonly known in the art of fragrance compounding. It is preferable to store the final sensory fragrance mixture under ambient conditions for a few hours after mixing before using it as a component of a personal care composition.

The personal care compositions useful in the methods of the invention may then be produced by blending the desired components with the sensory fragrance using equipment and methodology commonly known in the art of personal care product manufacture. In order to improve the solubilization of the sensory fragrance in aqueous personal care compositions, the sensory fragrance may be pre-blended with one or more of the nonionic surfactants.

Personal care compositions include personal cosmetic, toiletry, and healthcare products such as dry and wet wipes, washes, baths, shampoos, gels, soaps, sticks, balms, mousses, sprays, lotions, creams, cleansing compositions, powders, oils, bath oils and other bath compositions which may be added to a bath. The aforementioned wipes, washes, baths, shampoos, gels, soaps, sticks, balms, mousses, sprays, lotions, creams, cleansing compositions, oils and bath oils are commercially known to those who have a knowledge of preparing personal care compositions. Suitable personal care compositions include, but are not limited to, Johnson's Bedtime Bath® product. In order to achieve the desired response in a mammal, the personal care composition may be used in a dosing amount that is in accordance with the prescribed directions of the personal care composition.

Although a greater effect is generally achieved when multiple stimuli are used together, a single stimuli can also be effective so are included in the invention.

As discussed above, it has been discovered, that the administration of a sensory regimen can result in improved sleep behaviors and behaviors related to sleep of a mammal. In another embodiment of the invention, the combination of the use of the sensory regimen and the CRH antagonist provides for a more potent treatment. In another embodiment, the combination of the use of the sensory regimen and the CRH antagonist allows for a lower dose of the CRH antagonist to be used.

Examples of CRH antagonists include, but are not limited to, Astressin, D-PheCRH (12-41), and alpha helical CRH (9-41), and others known in the art. In yet another embodiment, the methods according to the invention may be practiced in combination with the administration of pharmacetucicals that reduce CRH, such as selective serotonin reuptake inhibitors (SSRI) including but not limited to antidepressants, such as, for example, Prozac. Such pharmaceuticals should be administered in accordance with the directions prescribed by an authorized physician.

In another embodiment, the methods of the invention may be practiced in combination with the administration of pharmaceutical or over-the-counter sleep medication. The negative side effects of pharmaceutical or over-the-counter sleep medication, such as risk of reliance and feeling sleepy the next morning, may be reduced if the dosage of the medication can be reduced. In yet another embodiment, the combination of the use of the sensory regimen and sleep medication, allows for a lower dose of sleep medication to be used.

To illustrate the methods of the invention, the following examples are included. These examples do not limit the invention. They are meant only to suggest a method of practicing the invention. Those knowledgeable in the calming of human beings as well as other specialties may find other methods of practicing the invention. Those methods are deemed to be within the scope of this invention.

### EXAMPLES

### Examples 1-3

Three groups of women (Groups A-C) participated in a study in which mood and sleep behavior self-assessments were made and saliva samples were collected at set time points throughout the day for the purpose of measuring cortisol.

In Example 1, Group A was exposed to a one time relaxing fragrance experience at a set point in the morning.

In Example 2, Group B was exposed to the same fragrance experience as in Group A but with multiple exposures through the day, including one prior to the onset of sleep.

In Example 3, Group C was exposed to the same fragrance as Groups A & B but was also exposed to relaxing music during the same period. Group C had multiple exposures to the music and fragrance at set time points throughout the day. At a set time prior to the anticipated onset of sleep, panelists in Group C bathed in a warm (about 33-37°C) tub with the same fragrance as experienced throughout the day, with music and low ambient lighting.

The fragrance and music stimuli used in Examples 2-6 were the same fragrance and music stimuli as used in Example 1.

### Example 1: One Time Exposure to Fragrance (Group A)

A group of women aged 20-40 years and in good health (Group A) participated in an ambulatory study in their natural environment in which they were asked to collect approximately 1ml of saliva by drooling or spitting into independent vials at set points throughout each day of the study for the purpose of measuring cortisol concentrations. These saliva samples were collected:
i) upon waking
ii) 30 minutes post waking
iii) 65 minutes post waking
iv) 4 hours post waking
v) 8 hours post waking
vi) 12 hours post waking.

The group of women was also asked to complete self-assessments of their mood and behavior. The study lasted for 5 days. Day 1 of the study served as the control day in which saliva samples were collected and questionnaires completed but no treatment regimen had been prescribed. On Day 2 of the study, the panelists were asked to smell a pleasant relaxing fragrance for a period of 5 minutes, which occurred approximately 30 minutes after morning waking. On days 2-5 no treatment regimen was prescribed.

Salivary samples were sent to Salimetrics, LLC, 1981 Pine Hall Rd, State College PA 16801 for the determination of concentration of cortisol. The group mean salivary cortisol concentrations each of these collection times is given in Table 1 below.

**Table 1**

| Minutes Since Morning Waking | Day 1 (µg/dl) | Day 2 (µg/dl) | Day 3 (µg/dl) | Day 4 (µg/dl) | Day 5 (µg/dl) |
|---|---|---|---|---|---|
| 30 | 0.484 | 0.511 | 0.436 | 0.416 | 0.598 |
| 240 | 0.157 | 0.145 | 0.16 | 0.134 | 0.262 |
| 480 | 0.137 | 0.147 | 0.214 | 0.138 | 0.128 |
| 720 | 0.186 | 0.072 | 0.15 | 0.087 | 0.097 |

An integrative measure of cortisol calculated from the area under the curve for each day of the study may be made. The values of the area under the curve (AUC) for Group A for each of the 5 days of the study are presented in Table 2 below.

**Table 2**

| Day | Total AUC (arbitrary units) |
|---|---|
| 1 | 130 |
| 2 | 160 |
| 3 | 150 |
| 4 | 120 |
| 5 | 160 |

The value of the AUC minus the peak area for Group A for each of the 5 days of the study is presented in Table 3 below.

**Table 3**

| Day | AUC Minus Peak Area (arbitrary units) |
|---|---|
| 1 | 110 |
| 2 | 90 |
| 3 | 120 |
| 4 | 90 |
| 5 | 130 |

The mean cortisol for Group A four hours post waking are presented in Table 4 below.

**Table 4**

| Day | Mean Cortisol 4 Hours Post Waking (µg/dl) |
|---|---|
| 1 | 0.157 |
| 2 | 0.145 |
| 3 | 0.160 |
| 4 | 0.134 |
| 5 | 0.262 |

### Example 2: Multiple Exposures to Pleasant Relaxing Fragrance and Ambient Lighting (Group B)

A group of women aged 20-40 years and in good health (Group B) participated in an ambulatory study in their natural environment in which they were asked to collect approximately 1ml of saliva by drooling or spitting into independent vials at set points throughout each day of the study for the purpose of measuring cortisol concentrations. These saliva samples were collected:
i) upon waking
ii) 30 minutes post waking
iii) 65 minutes post waking
iv) 4 hours post waking
v) 8 hours post waking
vi) 12 hours post waking.

They were also asked to complete self-assessments of their mood and sleep behavior. The study lasted for 5 days. Day 1 of the study served as the control day in which saliva samples were collected and questionnaires completed but no treatment regimen had been prescribed. On days 2-5 of the study, the panelists were asked to smell a pleasant relaxing fragrance while sitting in comfortably in a room with low level of ambient lighting for a period of 5 minutes approximately 30 minutes after morning waking, 4 hours after waking and 8 hours after waking.

Salivary samples were sent to Salimetrics, LLC, 1981 Pine Hall Rd, State College PA 16801 for the determination of concentration of cortisol. The group mean salivary cortisol concentrations each of these collection times is given in Table 4 below.

**Table 4**

| Minutes Since Morning Waking | Day 1 (µg/dl) | Day 2 (µg/dl) | Day 3 (µg/dl) | Day 4 (µg/dl) | Day 5 (µg/dl) |
|---|---|---|---|---|---|
| 30 | 0.362 | 0.391 | 0.336 | 0.434 | 0.483 |
| 240 | 0.111 | 0.262 | 0.309 | 0.303 | 0.183 |
| 480 | 0.063 | 0.266 | 0.389 | 0.204 | 0.307 |
| 720 | 0.038 | 0.058 | 0.085 | 0.098 | 0.194 |

For Group B, day 1 was the control, while on beginning on day 2 and continuing through day 5, the panelists were exposed to fragrance at 3 time points throughout the day. An integrative measure of cortisol calculated from the area under the curve for each day was made. The values of the area under the curve (AUC) for Group B for each of the 5 days of the study are presented in Table 5 below.

**Table 5**

| Day | Total AUC (arbitrary units) |
|---|---|
| 1 | 80 |
| 2 | 170 |
| 3 | 210 |
| 4 | 180 |
| 5 | 190 |

The value of the AUC minus the peak is for Group A for each of the 5 days of the study is presented in Table 6.

**Table 6**

| Day | AUC Minus Peak Area (arbitrary units) |
|---|---|
| 1 | 50 |
| 2 | 160 |
| 3 | 210 |
| 4 | 160 |
| 5 | 160 |

The mean cortisol for group B four hours post waking is shown in Table 7 below.

| Day | Mean Cortisol 4 Hours Post Waking (µg/dl) |
|---|---|
| 1 | 0.111 |
| 2 | 0.262 |
| 3 | 0.309 |
| 4 | 0.303 |
| 5 | 0.220 |

### Example 3: Multiple Exposures to Fragrance, Music and Ambient Lighting (Group C)

A group of women aged 20-40 years and in good health (Group C) participated in an ambulatory study in their natural environment in which they were asked to collect approximately 1 ml of saliva by drooling or spitting into independent vials at set points throughout each day of the study for the purpose of measuring cortisol concentrations. These saliva samples were collected:
i) upon waking
ii) 30 minutes post waking
iii) 65 minutes post waking
iv) 4 hours post waking
v) 8 hours post waking
vi) 12 hours post waking

They were also asked to complete self-assessments of their mood and sleep behavior. The study lasted for 5 days. Day 1 of the study served as the control day in which saliva samples were collected and questionnaires completed but no treatment regimen had been prescribed. On days 2-5 of the study, the panelists were asked to smell a pleasant relaxing fragrance and while sitting in comfortably in room with low ambient lighting and listening to relaxing music for a period of 5 minutes approximately 30 minutes after morning waking, 4 hours after waking and 8 hours after waking. Prior to bedtime on days 2-5 panelists were also asked to take a 15-minute fragrance bath at approximately 35°C while listening to relaxing music in a room with low ambient lighting.

Salivary samples were sent to Salimetrics, LLC, 1981 Pine Hall Rd, State College PA 16801 for the determination of concentration of cortisol. The group mean salivary cortisol concentrations each of these collection times is given in Table 8 below.

**Table 8**

| Minutes Since Morning Waking | Day 1 (µg/dl) | Day 2 (µg/dl) | Day 3 (µg/dl) | Day 4 (µg/dl) | Day 5 (µg/dl) |
|---|---|---|---|---|---|
| 30 | 0.518 | 0.402 | 0.331 | 0.355 | 0.389 |
| 240 | 0.233 | 0.219 | 0.167 | 0.139 | 0.186 |
| 480 | 0.138 | 0.144 | 0.125 | 0.110 | 0.116 |
| 720 | 0.056 | 0.094 | 0.140 | 0.065 | 0.060 |

Day 1 was the control, while on day 2 the panelist experienced fragrance, relaxing music and low ambient lighting at 3 time points throughout the day, and a bath with a relaxing fragrance coupled with relaxing music under low ambient lighting prior to bedtime, which would be repeated through and including Day 5. An integrative measure of cortisol calculated from the area under the curve for each day may be made. The values of the area under the curve (AUC) for Group C for each of the 5 days of the study are presented in Table 9 below.

**Table 9**

| Day | Total AUC (arbitrary units) |
|---|---|
| 1 | 150 |
| 2 | 140 |
| 3 | 120 |
| 4 | 100 |
| 5 | 120 |

The values of the AUC minus the morning peak area for Group C and the cortisol concentration 4 hours post waking for each of the 5 days of the study are present in Tables 10 and 11 respectively below.

**Table 10**

| Day | AUC Minus Peak Area (arbitrary units) |
|---|---|
| 1 | 120 |
| 2 | 120 |
| 3 | 100 |
| 4 | 80 |
| 5 | 100 |

**Table 11**

| Day | Mean Cortisol 4 Hours Post Waking (µg/dl) |
|---|---|
| 1 | 0.233 |
| 2 | 0.219 |
| 3 | 0.167 |
| 4 | 0.139 |
| 5 | 0.186 |

The cortisol data for Group C surprisingly indicates a reduction in cortisol for days 2-5 in comparison to control day 1. Importantly, a reduction in cortisol was found in all of the indices useful in studying HPA activity: total daily cortisol, cortisol minus the morning peak, and the cortisol value approximately 4 hours post waking. This clearly demonstrates that a combination or regimen of sensory stimuli can provide long term and lasting effects on sleep of the individual, by modifying HPA activity.

It is noted that, while the same relaxing fragrance was used throughout the three different cells, and provided a relaxing and pleasing sensation to Groups A and B, no long-lasting effect on sleep reduction as measured by any of the indices useful in studying HPA activity: total daily cortisol, cortisol minus the morning peak, and the cortisol value approximately 4 hours post waking was observed. These examples clearly demonstrate that there is a difference between a momentary, pleasing effect, and a long lasting effect that can reduce the activity of one's HPA axis.

### Example 4: Reduction of HPA Axis Activity Improves Sleep Behavior of Individuals

The St. Mary's Sleep Questionnaire, as described by T. J. Leigh, H. A. Bird, I. Hindmarch, P. D. Constable, V. Wright in "Factor analysis of the St. Mary's Hospital sleep questionnaire" *Sleep* 1988; 11: 448-453, is a self-assessment questionnaire used in the field of sleep research to quantitatively evaluate a range of sleep parameters. The questions and rating scales used in this questionnaire are given here:

*This questionnaire refers to your sleep over the past 24 hours. Please try to answer every question.*

*At what time did you:*
*1. Settle down for the night? Hour Minutes (am*/*pm)*
*2. Fall asleep last night? Hour Minutes (am*/*pm)*
*3. Finally wake this morning?* Hour Minutes
*4. Get up this morning?* Hour Minutes
*5. Was your sleep: (check below)* Hour Minutes
   *1. Very light*
   *2. Light*
   *3. Fairly Light*
   *4. Light average*
   *5. Deep average*
   *6. Fairly Deep*
   *7. Deep*
   *8. Very Deep*
*6. How many times did you* wake *up? (check below)*
   *0. Not at all*
   *1. Once*
   *2. Twice*
   *3. Three times*
   *4. Four times*
   *6. Six times*
   *7. More than six times*
   *How much sleep did you have:*
*7. Last Night?* *Hours* *Minutes*
*8. During the day, yesterday?* *Hours* *Minutes*
*9. How well did you sleep last night? (check below)*
   *1. Very badly*
   *2. Badly*
   *3. Fairly badly*
   *4. Fairly well*
   *5. Well*
   *6. Very well*
   *If not well, what was the trouble? (e.g., restless, etc)*
   *1.*
   *2.*
   *3.*
*10. How clear-headed did you feel after getting up this morning? (check below)*
   *1. Still very drowsy indeed*
   *3. Still moderately drowsy*
   *3. Still slightly drowsy*
   *4. Fairly clear-headed*
   *5. Alert*
   *6. Very alert*
*11. How satisfied were you with last night's sleep?*
   *1. Very unsatisfied*
   *2. Moderately unsatisfied*
   *3. Slightly unsatisfied*
   *4. Fairly satisfied*
   *5. Completely satisfied*
*12. Were you troubled by waking early and being unable to get off to sleep again?*
   *1. No*
   *2. Yes*
*13. How much difficulty did you have in getting off to sleep last night? (check below)*
   *1. None or very little*
   *2. Some*
   *3. A lot*
   *4. Extreme difficulty*
*14. How long did it take you to fall asleep last night?*
   *Hours* *Minutes*

Panelists rate each parameter on the given scale accordingly. Groups A, B and C from examples 1, 2 and 3 respectively, completed the St. Mary's Sleep questionnaire on days 2, 4 and 5 of the 5-day long study. The aim of the use of this questionnaire was to determine how the reduction of HPA activity induced by the treatment regimens affected the sleep behavior of the individuals participating in the study. The results are presented in Table 12 below, comparing the first and last days of the study period.

**Table 12**

| % Improvement | Group A | Group B | Group C |
|---|---|---|---|
| Sleep depth | -8 | 0 | 55 |
| Number of night awakenings | 0 | -200 | 50 |
| Total sleep time | 10 | 4 | 7 |
| Sleep quality | 3 | 4 | 37 |
| Alertness | 24 | 24 | 27 |
| Satisfaction | 8 | 22 | 31 |
| Early awakening | 27 | -10 | 25 |
| Difficulty going to sleep | -11 | -17 | 38 |
| Latency to sleep | 24 | 28 | 69 |

Overall, the results indicate that the sleep behavior of an individual may be improved by reduction of the HPA axis activity. Aspects of sleep behavior are most significantly improved for Group C. This observation is consistent with the reduction of HPA axis activity found for Group C.

Sleep quality is a composite of many parameters, including latency to sleep, difficulty going to sleep, number of night awakenings and others. As a composite parameter, sleep quality is a good overall indicator of sleep behavior when looking for changes in sleep behavior in a group of individuals, as aspects of sleep behavior vary from individual to individual. For example, one individual may report difficulty going to sleep whereas another individual may report experiencing night awakenings. In both cases, sleep quality is affected. Sleep quality was improved most significantly for Group C, which was the group for which a reduction in HPA axis activity was found.

### Example 5: Showering Prior to Bedtime (Group D) and Showering Prior to Bedtime with a Fragranced Shower Product (Group E)

Two groups of women aged 20-40 years and in good health (Groups D and E) participated in an ambulatory study in their natural environment in which they were asked to collect approximately 1ml of saliva by drooling or spitting into independent vials at set points throughout on the first and last days of the study for the purpose of measuring cortisol concentrations.

These saliva samples were collected:
i) 45 minutes prior to bedtime and immediately before showering; and
ii) immediately prior to bedtime.

They were also asked to complete self-assessments of their mood and sleep behavior using the St Mary's Sleep Questionnaire as previously described in Example 4. The study lasted for 3 days. Day 1 of the study served as the control day in which saliva samples were collected and questionnaires completed but no treatment regimen had been prescribed. On days 2 & 3 of the study, the panelists were asked to shower prior to bedtime. Group D showered without use of any products or stimuli in a shower at water temperature approximately 35°C; whereas Group E showered using a fragranced shower gel product in a shower at a water temperature of approximately 35°C prior to bedtime.

Salivary samples were sent to The Center for Psychobiological and Psychosomatic Research, Universitaetsring 15, D-54286, University of Trier, Germany, for the determination of concentration of cortisol.

In previous examples cortisol concentration was reported in micrograms/deciliter. The cortisol concentration data reported in examples 5-8 is reported in nanomoles/liter. For comparative purposes, 1 microgram/deciliter is equivalent to 27.6 nanomoles/liter.

**Table 13**

| Sample | Group D Day 1 | Group D Day 3 | Group E Day 1 | Group E Day 3 |
|---|---|---|---|---|
| Mean cortisol 45 minutes prior to bedtime (nmol/l) | 4.03 | 6.19 | 4.73 | 6.42 |
| Mean cortisol immediately prior to bedtime (nmol/l) | 4.56 | 5.75 | 5.26 | 6.10 |
| Delta Prior to Bedtime | 0.54 | -0.43 | 0.53 | -0.32 |

The Group D and Group E mean cortisol values on treatment days 3 indicate a group mean decrease in cortisol levels over the 45-minute period prior to bedtime. In comparison, on control day 1, the cortisol level does not decrease. These results indicate that the shower experience prior to bedtime helped to promote a reduction in cortisol.

Improvements in sleep behavior were observed for Groups D and E as reported in Table 14 below, comparing the first and last days of the study period. These sleep improvements are consistent with the observed reductions in cortisol. Both groups showed cortisol reductions after showering.

**Table 14**

| % Improvement | Group D | Group E |
|---|---|---|
| Sleep depth | 0 | 13 |
| Number of night awakenings | -20 | -51 |
| Total sleep time | 1 | 4 |
| Sleep quality | 10 | 14 |
| Alertness | -2 | 33 |
| Satisfaction | -2 | 12 |
| Early awakening | 4 | -6 |
| Difficulty going to sleep | 0 | -17 |
| Latency to sleep | -22 | -49 |

As was discussed in Example 4, many parameters can be used in self-assessment studies of sleep behavior, and sleep quality is a composite of many of these parameters, including latency to sleep, difficulty going to sleep, number of night awakenings and others. As a composite parameter, sleep quality is a good overall indicator of sleep behavior when looking for changes in sleep behavior in a group of individuals, as aspects of sleep behavior vary from individual to individual. For example, one individual may report difficulty going to sleep whereas another individual may report experiencing night awakenings. In both cases, sleep quality is affected.

The results in Table 14 indicate that aspects of sleep behavior were improved by showering before bedtime. Further, sleep quality was more significantly improved when sensory stimuli, presented in the form of a fragranced shower product in this example, was used in the showering experience.

### Example 6: Bathing prior to bedtime (Group F) and Bathing prior to bedtime with Bath Product (Group G) - Effect on HPA axis activity and Sleep behavior.

Two groups of women aged 20-40 years and in good health (Groups F and G) participated in an ambulatory study in their natural environment in which they were asked to collect approximately 1ml of saliva by drooling or spitting into independent vials at set points on the first and last days of the study for the purpose of measuring cortisol concentrations.

These saliva samples were collected:
i) 45 minutes prior to bedtime and immediately before bathing
ii) immediately prior to bedtime

They were also asked to complete self-assessments of their mood and sleep behavior using the St Mary's Sleep Questionnaire as previously described in Example 4. The study lasted for 3 days. Day 1 of the study served as the control day in which saliva samples were collected and questionnaires completed but no treatment regimen had been prescribed. On days 2 & 3 of the study, the panelists were asked to bathe prior to bedtime. Group F were asked to bathe without use of any products or stimuli in a bath at a water temperature of approximately 35°C whereas Group G were asked to bathe using a fragranced bubble bath product in a bath of water at a temperature of approximately 35°C immediately prior to bedtime.

Salivary samples were sent to The Center for Psychobiological and Psychosomatic Research, Universitaetsring 15, D-54286, University of Trier, Germany, for the determination of concentration of cortisol.

**Table 15**

| Sample | Group F Day 1 | Group F Day3 | Group G Day 1 | Group G Day 3 |
|---|---|---|---|---|
| Mean cortisol level 45 minutes prior to bedtime (nmol/l) | 4.46 | 5.51 | 6.79 | 7.77 |
| Mean cortisol level immediately prior to bedtime (nmol/l) | 5.56 | 5.87 | 6.13 | 7.20 |
| Delta Prior to Bedtime (nmol/l) | 1.10 | 0.36 | -0.66 | -0.57 |

The Group F mean cortisol values indicated a group mean increase in the 45 minutes prior to bedtime on all three days of the study. However the magnitude of the increase on the control day (day 1) was greater than the magnitude of the increases on the treatment day (day 3) of the study.

The Group G mean cortisol values on treatment day 3 indicates a decrease on day 3 in the 45-minute period preceding bedtime. A group mean cortisol decrease was also observed in the 45-minute period prior to bedtime on control day 1. These results indicate that the bathing experience with the fragranced bubble bath product prior to bedtime is supportive of a reduction in cortisol.

Improvements in sleep behavior were observed for Groups F and G as reported in Table 16 below. For Group G, these sleep improvements are consistent with the observed reductions in cortisol after bathing.

**Table 16**

| % Improvement | Group F | Group G |
|---|---|---|
| Sleep depth | 26 | 14 |
| Number of night awakenings | 25 | 75 |
| Total sleep time | 1 | -1 |
| Sleep quality | 4 | 13 |
| Alertness | 26 | 23 |
| Satisfaction | 9 | 5 |
| Early awakening | 13 | 21 |
| Difficulty going to sleep | 13 | -8 |
| Latency to sleep | 49 | -3 |

As in previous examples, the composite parameter, sleep quality serves as a good overall indicator of sleep behavior. The results in Table 16 indicate that aspects of sleep behavior are improved by bathing before bedtime. Further, sleep quality was more significantly improved when sensory stimuli, presented in the form of a fragranced bath product in this example, was used in the bathing experience.

### Example 7: Bathing prior to bedtime with a bath product in a dimly lit room while listening to music (Group H) - Effect on HPA axis activity and sleep behavior

A group of women aged 20-40 years and in good health (Group H) participated in an ambulatory study in their natural environment in which they were asked to collect approximately 1ml of saliva by drooling or spitting into independent vials at set points throughout each day of the study for the purpose of measuring cortisol concentrations.

These saliva samples were collected:
i) 180 minutes prior to bedtime
ii) 120 minutes prior to bedtime
iii) 45 minutes prior to bedtime and immediately before bathing
iv) immediately prior to bedtime

They were also asked to complete self-assessments of their mood and sleep behavior. The study lasted for 4 days. Day 1 of the study served as the control day in which saliva samples were collected and questionnaires completed but no treatment regimen had been prescribed. On days 2 - 4 of the study, the panelists were asked to bathe prior to bedtime for a period of 15 minutes in a fragrance bath at approximately 35C containing fragranced bubble bath product while listening to relaxing music in a room with low ambient lighting.

Salivary samples were sent to The Center for Psychobiological and Psychosomatic Research, Universitaetsring 15, D-54286, University of Trier, Germany, for the determination of concentration of cortisol. The group mean salivary cortisol concentrations each of these collection times is given in Table 17 below.

**Table 17**

| Minutes Before Bedtime | Day 1 (nmol/l) | Day 2 (nmol/l) | Day3 (nmol/l) | Day 4 (nmol/l) |
|---|---|---|---|---|
| 180 | 8.03 | 7.69 | 7.95 | 8.29 |
| 120 | 7.66 | 6.73 | 7.71 | 6.54 |
| 45 | 6.34 | 5.79 | 6.68 | 6.93 |
| 0 | 6.92 | 4.72 | 6.41 | 6.68 |

The values of the AUC are for Group H for each of the 4 days of the study and for the control and the treatment days are presented in Tables 18 and 19 below.

**Table 18**

| Day | Area under Curve (arbitrary units) |
|---|---|
| 1 | 1290 |
| 2 | 1140 |
| 3 | 1300 |
| 4 | 1260 |

**Table 19**

| Condition | Mean AUC (arbitrary units) |
|---|---|
| Control | 1290 |
| Sensory Treatment | 1230 |

The group mean values of cortisol before bedtime on each of the 4 days of the study are presented below in Table 20.

**Table 20**

| Sample | Day 1 (nmol/l) | Day 2 (nmol/l) | Day3 (nmol/l) | Day 4 (nmol/l) |
|---|---|---|---|---|
| Mean cortisol 45 minutes prior to bedtime (nmol/l) | 6.34 | 5.79 | 6.68 | 6.93 |
| Mean cortisol immediately prior to bedtime (nmol/l) | 6.92 | 4.72 | 6.41 | 6.68 |
| Delta | 0.58 | -1.07 | -0.27 | -0.25 |

The cortisol data in the 45 minutes before bedtime for Group H (Table 20) surprisingly indicates a reduction in cortisol for treatment days (2-4) in comparison to control day 1. Importantly, a reduction in cortisol was found in all of the indices useful in studying HPA axis activity in the hours preceding sleep: total cortisol in the 3 hours preceding bedtime, decreasing cortisol in the 45 minutes preceding bedtime and the cortisol value immediately before bedtime. This clearly demonstrates that a combination or regimen of sensory stimuli can provide long term and lasting effects on the HPA axis activity of an individual in relation to bedtime.

A comparison of the cortisol data in the 45 minutes prior to bedtime shows that while a single sensory stimulus can help promote cortisol reduction in the time prior to bedtime, use of combinations of sensory stimuli can have a more significant effect.

Improvements in sleep behavior were observed for Group H as reported in Table 21 below. These sleep improvements are consistent with the observed reductions in total cortisol in the 3 hours preceding bedtime, decreasing cortisol in the 45 minutes preceding bedtime and the cortisol value immediately before bedtime due to multi-sensory bathing.

**Table 21**

| % Improvement | Group H |
|---|---|
| Sleep depth | 23 |
| Number of night awakenings | 61 |
| Total sleep time | 1 |
| Sleep quality | 12 |
| Alertness | 12 |
| Satisfaction | 15 |
| Early awakening | 8 |
| Difficulty going to sleep | 21 |
| Latency to sleep | 35 |

The results in Table 21 indicate that all aspects of sleep behavior are improved for Group H. As in previous examples, the composite parameter, sleep quality serves as a good overall indicator of sleep behavior, and this parameter is improved by multi-sensory bathing.

Overall these results indicate that a reduction in basal cortisol levels prior to bedtime, a trend of decreasing cortisol levels in the 45 minutes or so preceding bedtime, reductions in total cortisol in the 2-3 hours preceding bedtime are all conducive to improved sleep behavior. Surprisingly, these effects on cortisol levels and the HPA axis can be delivered through use of sensory stimuli.

### Example 8: Resting prior to bedtime (Group I) - Effect on HPA axis activity and sleep behavior

A group of women aged 20-40 years and in good health (Group I) participated in an ambulatory study in their natural environment in which they were asked to collect approximately 1ml of saliva by drooling or spitting into independent vials at set points throughout each day of the study for the purpose of measuring cortisol concentrations.

These saliva samples were collected:
i) 240 minutes prior to bedtime
ii) 120 minutes prior to bedtime
iii) 45 minutes prior to bedtime and immediately before resting
iv) immediately prior to bedtime

They were also asked to complete self-assessments of their mood and sleep behavior. The study lasted for 5 days. Day 1 of the study served as the control day in which saliva samples were collected and questionnaires completed but no treatment regimen had been prescribed. On days 2 - 5 of the study, the panelists were asked to rest quietly for a period of 10 to 15 minutes beginning after collection of saliva sample iii of the evening.

Salivary samples were sent to The Center for Psychobiological and Psychosomatic Research, Universitaetsring 15, D-54286, University of Trier, Germany, for the determination of concentration of cortisol. The group mean salivary cortisol concentrations each of these collection times is given in Table 22 below.

**Table 22**

| Minutes Before Bedtime | Day 1 (nmol/l) | Day 2 (nmol/l) | Day3 (nmol/l) | Day 4 (nmol/l) |
|---|---|---|---|---|
| 240 | 4.87 | 4.99 | 4.9 | 5.76 |
| 120 | 4.34 | 3.77 | 4.25 | 5.21 |
| 45 | 4.46 | 4.73 | 4.48 | 5.17 |
| 0 | 3.69 | 4.03 | 3.88 | 4.58 |

The values of the AUC are for Group I for each of the 4 days of the study and for the control and the treatment days are presented in Tables 23 and 24 below.

**Table 23**

| Day | AUC (arbitrary units) |
|---|---|
| 1 | 1070 |
| 2 | 1040 |
| 3 | 1070 |
| 4 | 1270 |

**Table 24**

| Condition | Mean AUC (arbitrary units) |
|---|---|
| Control | 1070 |
| Resting | 1120 |

The group mean values of cortisol immediately before bedtime on each of the 4 days of the study are presented below in Table 25.

**Table 25**

| Sample | Day 1 (nmol/l) | Day 2 (nmol/l) | Day3 (nmol/l) | Day 4 (nmol/l) |
|---|---|---|---|---|
| Mean cortisol 45 minutes prior to bedtime (nmol/l) | 4.46 | 4.73 | 4.48 | 5.17 |
| Mean cortisol immediately prior to bedtime (nmol/l) | 3.69 | 4.03 | 3.88 | 4.58 |
| Delta | -0.77 | -0.7 | -0.6 | -0.59 |

The cortisol data in the 45 minutes before bedtime for Group I (table 25) indicate a reduction in cortisol in the 45 minutes prior to bedtime on all 4 days of the study, as would be physiologically desirable at this time of day and is expected in mammals exhibiting a diurnal rhythm for cortisol secretion. Importantly, however no consistent trend of reduction in cortisol was found on treatment days, as compared to the control day, or in the other indices useful in studying HPA activity in the hours preceding sleep: total cortisol in the 3-4 hours preceding bedtime, and the cortisol value immediately before bedtime. This clearly demonstrates that while resting prior to bedtime is not inconsistent with the natural diurnal rhythm for cortisol secretion it does not downregulate basal antivity of the HPA axis in the manner that has been surprisingly found through use of a combination or regimen of Sensory stimuli.

Improvements in most of the aspects sleep behavior were observed for Group I as reported in Table 26 below.

**Table 26**

| % Improvement | Group I |
|---|---|
| Sleep depth | 7 |
| Number of night awakenings | 39 |
| Total sleep time | 0 |
| Sleep quality | 2 |
| Alertness | 4 |
| Satisfaction | -2 |
| Early awakening | 4 |
| Difficulty going to sleep | 17 |
| Latency to sleep | 11 |

As in previous examples, the composite parameter, sleep quality serves as a good overall indicator of sleep behavior, and this parameter is only marginally improved by resting before bedtime.

That a significant improvement in sleep quality was not found by resting before bedtime, is consistent with the observation that there was no downregulation of HPA activity attributable to resting before bedtime.

## Claims

1. A method of affecting sleep and sleep-related behaviors in a mammal having a diurnal rhythm, comprising the step of:
reducing the basal activity of the HPA axis of said mammal;
wherein said HPA axis, comprises:
a. levels of adrenocortical hormone present as a function of time in said diurnal rhythm of said mammal;
b. a total daily amount of adrenocortical hormone;
c. an integrative measure of morning peak adrenocortical hormone; and
d. an onset of sleep threshold.

2. A method of affecting sleep and sleep-related behaviors in a mammal having a diurnal rhythm, comprising the step of:
reducing the basal activity of the HPA axis of said mammal,
wherein said reducing step, comprises administering an effective amount of a sensory regimen to said mammal; and
wherein said HPA axis, comprises:
a. level of adrenocortical hormone present as a function of time in said diurnal rhythm of said mammal;
b. a total daily amount of adrenocortical hormone;
c. an integrative measure of morning peak adrenocortical hormone; and
d. an onset of sleep threshold.

3. The method of claim 1 or claim 2, wherein said reducing step is at least one step selected from the group consisting of:
a. reducing said average total daily amount of adrenocortical hormone in said mammal; and
b. reducing said average total daily amount adrenocortical hormone minus said integrative measure of morning peak adrenocortical hormone in said mammal.

4. A method of affecting sleep and sleep-related behaviors in a mammal having a diurnal rhythm, comprising at least one step selected from the group consisting of:
a. reducing the level of adrenocortical hormone in said mammal 4 hours to 8 hours after waking;
b. reducing the level of adrenocortical hormone in said mammal in the period of time preceding bedtime; and
c. reducing the level of adrenocortical hormone in said mammal below said onset of sleep threshold.

5. The method of claim 1 or claim 2, wherein said reducing step further comprises at least one step selected the group consisting of:
a. reducing said level of adrenocortical hormone in said mammal 4 hours to 8 hours after waking;
b. reducing said level of adrenocortical hormone in said mammal in the period of time preceding bedtime; and
c. reducing the level of adrenocortical hormone in said mammal below said onset of sleep threshold.

6. The method of claim 1 or claim 2, wherein said adrenocortical hormone is cortisol.

7. The method of claim 3, wherein said average total daily amount is reduced by at least about 5% to about 50%, based on the total daily amount of adrenocortical hormone present in said mammal at the start of said regimen.

8. The method of claim 3, wherein said average total daily amount is reduced by at least about 10% to about 40%, based on the total daily amount of adrenocortical hormone present in said mammal at the start of said regimen.

9. The method of claim 3, wherein said average total daily amount is reduced by at least about 15% to about 30%, based on the total daily amount of adrenocortical hormone present in said mammal at the start of said regimen.

10. The method of claim 3, wherein said average total daily amount of adrenocortical hormone minus said integrative measure of morning peak adrenocortical hormone is reduced by at least about 5% to about 70%, based on the total daily amount of adrenocortical hormone minus said integrative measure of morning peak adrenocortical hormone present in said mammal at the start of said regimen.

11. The method of claim 3, wherein said average total daily amount of adrenocortical hormone minus said integrative measure of morning peak adrenocortical hormone is reduced by at least about 10% to about 60%, based on the total daily amount of adrenocortical hormone minus said integrative measure of morning peak adrenocortical hormone present in said mammal at the start of said regimen.

12. The method of claim 3, wherein said average total daily amount of adrenocortical hormone minus said integrative measure of morning peak adrenocortical hormone is reduced by at least about 20% to about 50%, based on the total daily amount of adrenocortical hormone minus said integrative measure of morning peak adrenocortical hormone present in said mammal at the start of said regimen.

13. The method of claim 4, wherein said level of adrenocortical hormone 4 hours to 8 hours after waking is reduced by at least about 5% to about 70%, based on the level of adrenocortical hormone present during that same time period in the mammal at the start of the regimen.

14. The method of claim 5, wherein said level of adrenocortical hormone 4 hours to 8 hours after waking is reduced by at least about 5% to about 70%, based on the level of adrenocortical hormone present during that same time period in the mammal at the start of the regimen.

15. The method of claim 4, wherein said level of adrenocortical hormone 4 hours to 8 hours after waking is reduced by at least about 10% to about 60%, based on the level of adrenocortical hormone present during that same time period in the mammal at the start of the regimen.

16. The method of claim 5, wherein said level of adrenocortical hormone 4 hours to 8 hours after waking is reduced by at least about 10% to about 60%, based on the level of adrenocortical hormone present during that same time period in the mammal at the start of the regimen.

17. The method of claim 4, wherein said level of adrenocortical hormone 4 hours to 8 hours after waking is reduced by at least about 20% to about 50%, based on the level of adrenocortical hormone present during that same time period in the mammal at the start of the regimen.

18. The method of claim 5, wherein said level of adrenocortical hormone 4 hours to 8 hours after waking is reduced by at least about 20% to about 50%, based on the level of adrenocortical hormone present during that same time period in the mammal at the start of the regimen.

19. The method of claim 4, wherein said level of adrenocortical hormone preceding bedtime is reduced by at least about 3% to about 50%, based on the level of adrenocortical hormone present during that same time period in the mammal at the start of the regimen.

20. The method of claim 5, wherein said level of adrenocortical hormone preceding bedtime is reduced by at least about 3% to about 50%, based on the level of adrenocortical hormone present during that same time period in the mammal at the start of the regimen.

21. The method of claim 4, wherein said level of adrenocortical hormone preceding bedtime is reduced by at least about 5% to about 30%, based on the level of adrenocortical hormone present during that same time period in the mammal at the start of the regimen.

22. The method of claim 5, wherein said level of adrenocortical hormone preceding bedtime is reduced by at least about 5% to about 30%, based on the level of adrenocortical hormone present during that same time period in the mammal at the start of the regimen.

23. The method of claim 4, wherein said level of adrenocortical hormone preceding bedtime is reduced by at least about 5% to about 20%, based on the level of adrenocortical hormone present during that same time period in the mammal at the start of the regimen.

24. The method of claim 5, wherein said level of adrenocortical hormone preceding bedtime is reduced by at least about 5% to about 20%, based on the level of adrenocortical hormone present during that same time period in the mammal at the start of the regimen.

25. The method of claim 4, wherein said level of adrenocortical hormone is reduced to less than 0.3 micrograms/deciliter.

26. The method of claim 5, wherein said level of adrenocortical hormone is reduced to less than 0.3 micrograms/deciliter.

27. The method of claim 4, wherein said level of adrenocortical hormone is reduced to less than 0.2 micrograms/deciliter.

28. The method of claim 5, wherein said level of adrenocortical hormone is reduced to less than 0.2 micrograms/deciliter.

29. The method of claim 4, wherein said level of adrenocortical hormone is reduced to less than 0.15 micrograms/deciliter.

30. The method of claim 5, wherein said level of adrenocortical hormone is reduced to less than 0.15 micrograms/deciliter.

31. The method of claim 2, wherein said sensory regimen further comprises at least one step selected from the group consisting of:
a. administering at least one CRH antagonist;
b. administering at least one anti-depressant; and
c. administering at least one pharmacalogical sleep aid.

32. The method of claim 1 or claim 2, wherein basal activity of the HPA axis of said mammal is reduced within a period of 2 days to 14 days from the start of said regimen.

33. The method of claim 2, wherein said sensory regimen is selected from the group consisting of auditory stimuli, visual stimuli, tactile stimuli, gustatory stimuli and olfactory stimuli, and combinations thereof.

34. The use of a CRH antagonist in the manufacture of a medicament for affecting sleep and sleep-related behaviours in a mammal having a diurnal rhythm.
